Europäisches Patentamt

European Patent Office

Office européen des brevets

(11) Publication number: **0 298 389 B1**

# EUROPEAN PATENT SPECIFICATION

(45) Date of publication of patent specification: **07.10.92**

(51) Int. Cl.⁵: **A61M 15/00, A61M 16/00**

(21) Application number: **88110538.1**

(22) Date of filing: **01.07.88**

(54) **Intermittent signal actuated nebulizer.**

(30) Priority: **08.07.87 US 71202**

(43) Date of publication of application:
**11.01.89 Bulletin 89/02**

(45) Publication of the grant of the patent:
**07.10.92 Bulletin 92/41**

(84) Designated Contracting States:
**AT BE CH DE ES FR GB GR IT LI LU NL SE**

(56) References cited:
**WO-A-84/02656**
**DE-A- 2 809 255**
**DE-A- 3 000 222**
**DE-A- 3 008 406**
**US-A- 4 279 250**

(73) Proprietor: **VORTRAN MEDICAL TECHNOL-
OGY, INC.**
**3941 J. Street, Suite 354**
**Sacramento California 95819-3633(US)**

(72) Inventor: **Raabe, Otto G.**
**652 Buchanan Street**
**Davis, California 95616(US)**
Inventor: **Lee, James I.C.**
**2011 Wright Street, 15**
**Sacramento, California 95825(US)**

(74) Representative: **UEXKÜLL & STOLBERG Paten-
tanwälte**
**Beselerstrasse 4**
**W-2000 Hamburg 52(DE)**

Rank Xerox (UK) Business Services

## Description

### Technical Field

The present invention relates to nebulizers and to a corresponding method for creating medicinal aerosols for inhalation therapy as in the preamble of Claims 1 and 12, respectively. Such apparatuses and methods are known from either of WO 84/02656 or DE-2 809 255. In particular, the present invention relates to nebulizers used in conjunction with mechanical breathing machines which are used to ventilate the lungs of patients who cannot breathe unaided.

### Background Art

The thin membrane of the lungs provides an easily penetrated, convenient and generally safe means for obtaining rapid absorption of medication by the body. This is especially desirable where the lungs themselves are diseased or injured. Such medication or drugs are generally delivered to the lung membrane in the form of a fine mist or aerosol which is breathed into the lungs through the nose or mouth of the patient. A variety of devices, called nebulizers by those skilled in the art, have been developed for converting liquids into fine aerosols for this purpose. See, for example, WOA 8402656 (ETELA-HAMEEN KEUHKOVA-MMAYHDISTYS); DE-A-3008406 (VEB KOMINAT MEDIZIN UND LABORTECHNIK); US-A-4279250 (VALENTA et al.); DE-A-2809255 (ROSENTHAL); DE-A-3000222 (HAKKINEN). The simplest of these devices is the hand-held atomizer which converts a liquid to an aerosol when a bulb is compressed to produce a jet of air which atomizes the medication and propels it out of the atomizer. To be effective, the aerosols need to be provided at high concentrations and with droplet size in the respirable range (mass median aerodynamic diameter less than 5 micrometers).

Nebulizers are particularly useful for initiating and continuing respiratory therapy in conjunction with respirators, mechanical ventilators or breathing machines (hereinafter referred to generically as respirators) used to ventilate the lungs of patients having serious respiratory impairment. While some respirators incorporate nebulizers in their design, many do not. Nebulizers incorporated into the structure of such respirators often suffer from many disadvantages. One such disadvantage is severely limited capacity for medication to be nebulized, requiring frequent interruptions in the therapy as new medication is added to the nebulizer reservoir.

Another apparent disadvantage in such existing systems is the lack of a positive means for stirring the medication. This is particularly important to prevent settling when the liquid medication is a suspension. However, such stirring must not be so violent as to create turbulence capable of preventing or destroying nebulization.

Finally, some nebulizers are designed to operate continuously. Obviously this wastes both high pressure gas and medication, since the patient receives a benefit only during the inhalation phase. Those nebulizers which are designed to operate intermittently, i.e. only during the inhalation phase, are generally triggered by the movement of gas through the respirator during inhalation. This results in a slight delay in delivering medication to the patient, since some non-medicated gas will pass into the lungs before the nebulizer begins to operate. Thus, the patient does not receive the maximum possible amount of medication during the inhalation phase.

Most, if not all, such nebulizers are incorporated in respirators in which the inhalation and exhalation phases of the breathing cycle are triggered by changes in air pressure caused by the patient himself. Such "demand" respirators are not useful for patients whose respiratory systems are paralyzed and incapable of causing even slight changes in air pressure. These patients are aided by mechanical respirators in which the phases of the breathing cycle are triggered by electrical signals. There is now no convenient means of treatment for patients on such respirators.

Thus, the need exists for a nebulizer which can be attached to a mechanical respirator, especially those in which the breathing cycle is controlled by an electrical signal, which has a reservoir capacity sufficient to enable several hours of continuous treatment, which can prevent the settling of suspensions or mixtures without creating nebulization-destroying turbulence, and which can deliver medication to the patient just as the inhalation phase of the breathing cycle begins to insure that the patient will receive a maximum amount of medication during the inhalation phase without undue waste.

### Summary of the Invention

The present invention provides an apparatus for synchronizing the nebulizing of a liquid medicine with an external electrical signal source capable of generating a first and a second electrical signal, said apparatus comprising:

a housing containing a reservoir for holding liquid medicine to be nebulized;

an aerosol transducer for nebulizing said liquid medicine;

a single source of compressed gas; and,

a conduit for connecting the compressed gas to the aerosol transducer;

said aerosol transducer being connected by conduit to an electrical solenoid valve which is attached to compressed gas conduit;

said solenoid valve having two positions, the first position for connecting the compressed gas conduit and the aerosol transducer through conduit and the second position for connecting the compressed gas conduit and a turbine driven mixer (16) through a conduit (10) for mixing the liquid medicine to be nebulized, said solenoid valve being attached to said external electrical signal source for receiving the signal generated and synchronized for moving to the first position when the first electrical signal is received and for moving to the second position when the second electrical signal is received.

## Brief Description of the Drawings

A better understanding of the invention and its advantages will be apparent from the detailed description taken in conjunction with the accompanying drawings in which:

Figure 1 is a schematic side view a nebulizer of the present invention operationally attached to a mechanical respirator;

Figure 2 is a sectional side view of a nebulizer of the present invention;

Figure 3 is a perspective top external view of a nebulizer of the present invention attached to an external power source and signal generator; and,

Figure 4 is a sectional, perspective top view of the nebulizer of Figure 3.

## Detailed Description of the Invention

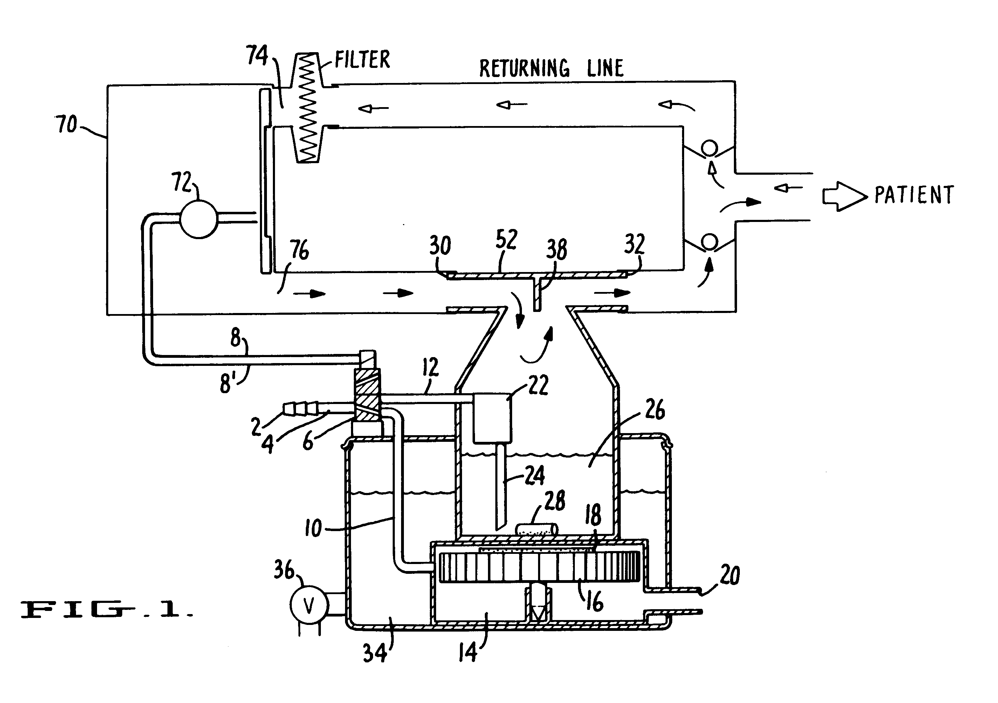

Figure 1 shows a nebulizer of the present invention operably connected to a mechanical respirator 70. The nebulizer comprises, in a housing, compressed gas inlet 2, at one end of a compressed gas conduit 4, adapted to be connected to a compressed gas source. Preferably this compressed gas source is the same source which is furnishing oxygen-enriched air to the respirator, and provides compressed air or oxygen mixture to the nebulizer ranging up to about 345 kPA (50 psig).

Compressed gas conduit 4 is connected at the other end to electrically operated nebulizer valve 6. Examples of such valves which have been found useful include the Honeywell® Skinner® K4M ultraminiature 4-way solenoid operated pneumatic valve and Numatic® Mark 3 solenoid operated valves.

Nebulizer valve 6 is connected by electrical lead wires 8, 8' to a signal source 72 on the respirator which controls the inhalation phase of the breathing cycle. Examples of such signal sources include a respirator solenoid, such as a solenoid actuated inhalation valve, an external electronic monitoring system, or an electronic interface attached to a signal generator on respirator 70, such as an interface connected to a logic circuit in the respirator. The key criteria here is to select a signal source which is synchronized with the breathing cycle of the respirator.

Nebulizer valve 6 also provides the conduits connecting the compressed gas source to turbine conduit 10 and to nebulizer conduit 12. Nebulizer valve 6 switches between two positions as electrical on/off signals are received. In the first position, during inhalation when the electric signal is "on", a passageway is opened between compressed gas conduit 4 and nebulizer conduit 12, and turbine conduit 10 is sealed off. In the second position, during exhalation when the electric signal is "off", a passageway is opened between compressed gas conduit 4 and turbine conduit 10, and nebulizer conduit 12 is sealed off. If desired, the effect of these signals could be reversed.

Turbine conduit 10 terminates, at its other end, in one end of chamber 14 which houses a rotating turbine 16 on top of which is mounted magnet 18. At the other end of chamber 14 is an exhaust conduit 20.

Nebulizer conduit 12 is attached, at its other end, to aerosol transducer 22, which includes liquid feed tube 24 extending into reservoir 26. Reservoir 26 includes magnetic stirring bar 28 which is located in the bottom of the reservoir. The liquid medicine contained in reservoir 26 is preferably kept at constant temperature by temperature bath 34. Alternatively, bath 34 may be changed or drained through spigot 36. The nebulizer is attached to respirator 70 at respirator input conduit 30 and at nebulizer output conduit 32.

The operation of this nebulizer in conjunction with a mechanical respirator 70 is illustrative of the many advantages it has over prior art nebulizers. Compressed gas, ranging up to about 345 kPA (50 psig), is provided continuously to the nebulizer unit through compressed gas inlet 2. This compressed gas may be premixed and enriched with oxygen.

When mechanical respirator 70 begins the inhalation phase of the respiratory cycle, signal source 72 switches to the "on" position as the respirator exhalation port 74 closes and the respirator inhalation port 76 opens. This signal is simultaneously picked up by nebulizer valve 6, which is attached to signal source 72 by electrical leadwires 8, 8′, and switches nebulizer valve 6 to the first position, opening a passageway between compressed gas conduit 4 and nebulizer conduit 12. The flow of compressed gas through turbine conduit 10 into chamber 14 ceases, stopping the stirring action of magnetic stirring bar 28 in reservoir 26. Compressed gas flows through compressed gas conduit 4, nebulizer conduit 12 and into aerosol transducer 22 which converts liquid medicine in reservoir 26 into an aerosol having droplets with a mass median aerodynamic diameter less than about 5 micrometers. The aerosol is generated into the airspace above reservoir 26. At essentially the same time, compressed gas passes into the respirator and enters the nebulizer through respirator input 30. Immediately upon entering the nebulizer, the compressed gas stream encounters wall 38 which deflects the air stream down into the airspace above reservoir 26, where it picks up the aerosol droplets and carries them through nebulizer output 32 and back into the mechanical respirator, and finally into the patient. Thus, even the air which initially enters the patient at the start of the inhalation phase will pass through the nebulizer and carry the medicinal aerosol.

When mechanical respirator 70 begins the exhalation phase of the respiratory cycle, signal source 72 switches to an "off" position, the respirator inhalation port 76 closes, and the respirator exhalation port 74 opens. Simultaneously, this signal is received by nebulizer valve 6 through electrical leadwires 8, 8', and the nebulizer valve 6 switches to its second ("off") position, opening a passageway between compressed gas conduit 4 and turbine conduit 10. The compressed gas enters chamber 14 where it encounters the fins or vanes attached to air-driven turbine 16 and moves them, rotating turbine 16. On top of rotating turbine 16 is mounted magnet 18 which exerts a magnetic force, causing magnetic stirring bar 28 in reservoir 26 to spin, stirring the liquid in reservoir 26. The compressed gas flows out of chamber 14 to the atmosphere through exhaust conduit 20.

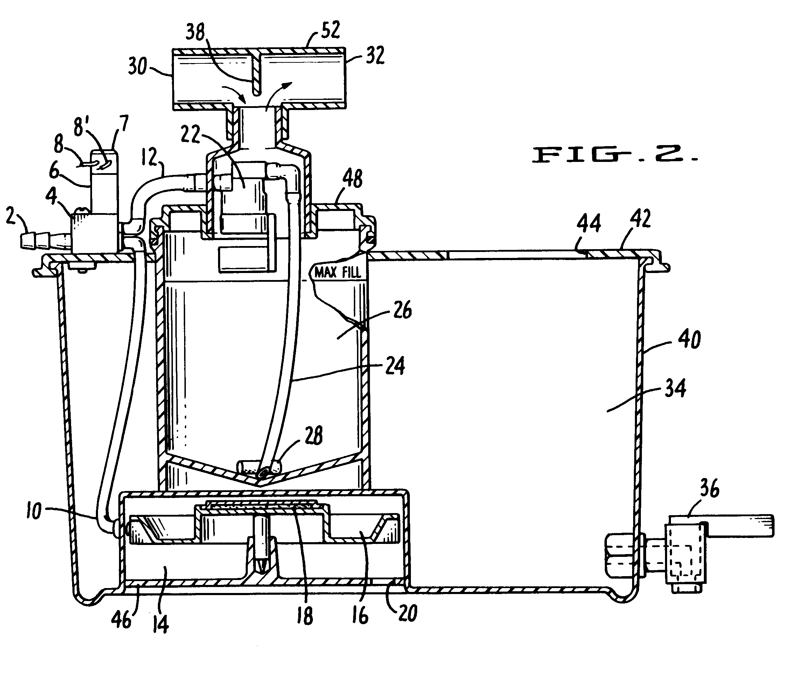

Figure 2 shows a cross-sectional view of a nebulizer of the present invention. Housing 40 can be constructed from plastic, metal or any other material suitable for holding a liquid temperature bath. Temperature bath 34 can be either cold or hot. Many methods are known for bringing such baths to and maintaining them at generally constant temperatures by those skilled in the art. For example, by using an ice water bath, the medicine in reservoir 26 can be maintained at 32 degree F (0 degrees C). A temperature bath 34 having a temperature greater than ambient air temperature can be obtained by applying sufficient heat to the exterior surface of housing 40 or by inserting a thermostatically controlled heating element through opening 44 in housing cover 42 to bring the liquid bath to and maintain it at the desired temperature.

A means for draining temperature bath 34 is preferably provided. This can be accomplished in many ways. One such way is shown in Figure 2 where a rotatable spigot 36 is provided in the bottom of housing 40 such that when spigot 36 is in an open position, a passageway to the outside of housing 40 is opened, permitting an liquid in temperature bath 34 to drain, and such that when spigot 36 is in a closed position, the passageway to the outside of housing 40 is sealed off.

Chamber 14 may be incorporated into a hollow recess in the bottom of housing 40. In this embodiment, air turbine 16 can be rotatably mounted on plate 46 which is attached to the sides of the hollow recess as shown in Figure 2. Magnet 18 may be attached to the top of turbine 16, or may be molded into the top of turbine 16 as shown in Figure 2. In this embodiment, when compressed gas is delivered to turbine conduit 10, the gas enters chamber 14 and encounters the vanes of air turbine 16, and causes the turbine 16 and the magnet 18 to rotate. The compressed gas stream then exits chamber 14 in any one of many ways. For example, plate 46 may have a single exhaust conduit 20, as shown in Figure 1 and 2, or may be perforated to permit the exhaust of the compressed gas stream from chamber 14.

As noted in the description of Figure 1 above, compressed gas is continuously supplied to nebulizer valve 6 through compressed gas inlet 2 and compressed gas conduit 4. Compressed gas conduit 4 ends at nebulizer valve 6, which contains a connector 7 for receiving electrical leadwires 8, 8' from a respirator signal source. Nebulizer valve 6 switches between two positions as signals are received from the respirator signal source through leadwires 8, 8'. In the first position, nebulizer valve 6 opens a passageway between compressed gas conduit 4 and nebulizer conduit 12 and seals off turbine conduit 10. In the second position, nebulizer valve 6 opens a passageway between compressed gas conduit 4 and turbine conduit 10, sealing off nebulizer conduit 12.

Turbine conduit 10 and nebulizer conduit 12 can be constructed of any material capable of channeling compressed gas. Preferably, however, they will be constructed from plastic tubing which is removably attached to permit easy cleaning and sterilization of the parts of the nebulizer unit after use, and to permit the removal of reservoir cover 48 to allow access to the interior of the reservoir. Many ways are known by those skilled in the art for providing such removable attachments. For example, a nipple, like that shown for the compressed gas inlet 2 in Figure 2, having an outside diameter slightly larger than the inside diameter of the plastic tubing used to form the conduits can be used. Because the wall of the plastic tubing is somewhat elastic, it can be forced over the end of such a nipple and will be frictionally held in position on the nipple until a sufficient force is exerted to pull it off the nipple. Generally, the force exerted by the compressed gas in the range used in this unit will not be sufficient to detach the tubing. However, a person pulling on the tubing can easily detach and reattach the tubing. Such a removable attachment can be used in any desired location.

Reservoir 26 is contained within housing 40 in a separate, and preferably removable, container. Reservoir 26 can be constructed of any material suitable for holding and dispensing medicine, such as plastic, stainless steel or glass. Further the reservoir may be constructed to be sterilizable, and hence reusable, or constructed to be disposable after one use. The size of reservoir 26 is limited only by the size of the housing 40. Preferably, reservoir 26 is of a size capable of holding at least 250 ml of liquid. This size permits up to 6 hours of operation before refilling or replacement of medication is necessary. Finally, the bottom of reservoir 26 may be sloped slightly to permit liquid feed tube 24 to drain essentially all of the liquid medication during use.

Reservoir cover 48 is removably attached to the top of reservoir 26 to seal the reservoir off from the atmosphere during operation of the nebulizer unit, to allow access to reservoir 26, and to provide a means for attaching the nebulizer unit to the respirator. Thus, the respirator input 30, nebulizer output 32, and wall 38 can be conveniently provided in respirator adapter 52 which can be integral with or removably attached to reservoir cover 48. Aerosol transducer 22 with attached liquid feed tube 24 are preferably attached to reservoir cover 48.

Magnetic stirring bar 28 may be of any size or material which will cooperate with the force exerted by magnet 18 to provide a stirring action when turbine 16 is rotated by compressed gas. Magnetic stirring bar 28 is preferably coated with an inert coating, such as Teflon, which permits easy sterilization and avoids any reaction with the liquid medicine.

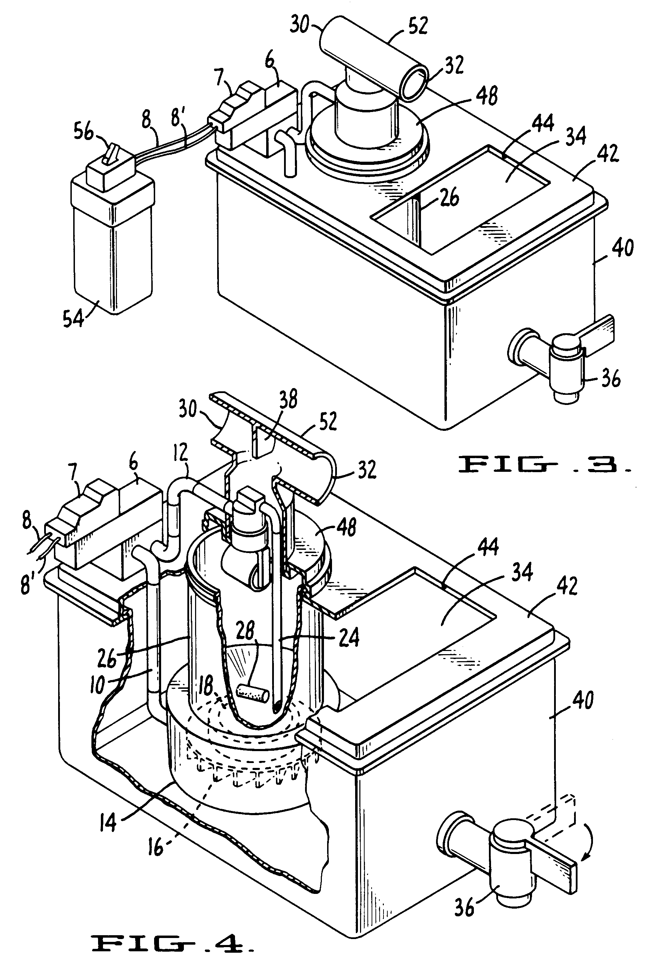

Figures 3 and 4 show a perspective exterior and sectional view of a nebulizer unit essentially as described above in Figure 2. Figure 3 shows spigot 36 in the closed position. In this position, the passageway from the inside of housing 40 to the outside through spigot 36 is closed and any liquid in temperature bath 34 cannot drain from housing 40. Figure 4 shows spigot 36 in the open position. In this position, the passageway from the inside of housing 40 to the outside through spigot 36 is open and any liquid in temperature bath 34 can drain from housing 40.

The nebulizer unit is attached to an existing respirator by connecting respirator adapter 52 to the respirator hose carrying compressed air and/or oxygen mixture to the patient, and by tapping signal source on the respirator which is synchronized to the breathing cycle using electrical lead wires 8, 8'. When the nebulizer is thus used with a mechanical respirator, no other external power is required. Thus, when the signal source is "off", nebulizer valve 6 switches to the second position and a passageway is opened allowing compressed gas to flow through turbine conduit 10. As discussed above, this rotates an air turbine mounted magnet in chamber 14, causing magnetic stirring bar 28 to spin, mixing the liquid in reservoir 26. This built-in mixing capability provides uniform nebulization of, for example, suspensions, colloids and liposomes in aqueous preparations over extended periods. While other mixing means are known and can be used, magnetic mixing as disclosed herein is preferred because the compressed gas can be used in conjunction with an air turbine, eliminating the need for an external power supply for the mixing function. Further, magnetic mixing is preferred because it thoroughly mixes without causing potential nebulization-destroying turbulence which may result when compressed gas is used directly to agitate the solution.

When the signal source is "on", nebulizer valve 6 switches to its first position, closing the passageway to turbine conduit 10 and opening the passageway to nebulizer conduit 12. This allows compressed gas to flow into aerosol transducer 22, nebulizing the liquid being drawn up through liquid feed tube 24 by Venturi vacuum, and filling the upper regions of reservoir cover 48 with the aerosol. As compressed gas passes into the nebulizer unit through respirator input 30, it is deflected downward by wall 38, picks up the aerosol and exits through nebulizer output 32, where it passes back into the respirator and is inhaled by the patient.

As shown in Figure 3, it is also possible to adapt the present invention to manual use with or without a respirator, by attaching electrical lead wires 8, 8' to a means for generating electrical signals, such as a battery 54 and switch 56. In this embodiment, a technician or a user can initiate nebulization by placing switch 56 in an "on" position. In this embodiment, the nebulizer may continuously nebulize the liquid medication in reservoir 26 until switch 56 is placed in an "off" position. Obviously, an external power source having appropriate voltage will work in conjunction with a switch. Where the external voltage is not appropriate, an interface which will step down or step up the voltage to an appropriate level can be used. However, a battery is most useful in situations where portability is important or where an appropriate external source of power is not available.

One skilled in the art will recognize at once that it would be possible to construct the various components of the present invention from a variety of materials. While the preferred embodiment has been described in detail and shown in the accompanying drawings, it will be evident that various further modifications are possible without departing from the scope of the invention as embodied in the claims.

## Claims

1. An apparatus for synchronizing the nebulizing of a liquid medicine with an external electrical signal source capable of generating a first and a second electrical signal, said apparatus comprising:

    a housing (40) containing a reservoir (26) for holding liquid medicine to be nebulized;

    an aerosol transducer (22) for nebulizing said liquid medicine;

    a single source of compressed gas; and,

    a conduit (12) for connecting the compressed gas to the aerosol transducer (22);

    said aerosol transducer (22) being connected by conduit (12) to an electrical solenoid valve (6) which is attached to compressed gas conduit (4);

    said solenoid valve (6) having two positions, the first position for connecting the compressed gas conduit (4) and the aerosol transducer (22) through conduit (12), said solenoid valve (6) being attached to said external electrical signal source (72) for receiving the signal generated and synchronized for moving to the first position when the first electrical signal is received and for moving to the second position when the second electrical signal is received, **characterized in that**

    the second position of said solenoid valve (6) is for connecting the compressed gas conduit (4) and a turbine driven mixer (16) through a conduit (10) for mixing the liquid medicine to be nebulized.

2. An apparatus according to claim 1 in which the first electrical signal is "on" and the second electrical signal is "off".

3. An apparatus according to claim 1 in which the first electrical signal is "off" and the second electrical signal is "on".

4. An apparatus according to claim 1 additionally wherein the turbine driven mixer (16) is attached to the housing outside of said reservoir (26) for mixing the liquid medicine to be nebulized.

5. An apparatus according to claim 4 in which said mixer (16) additionally includes a movable magnet (18) attached to said turbine (16), and a magnetic stirring bar (28) located inside said reservoir which cooperates with and is moved by the movement of said movable magnet (18).

6. An apparatus according to claim 1 in which said compressed gas ranges in pressure up to about 345 kPA (50 psig).

7. An apparatus according to claim 1 in which said electrical signal source comprises a battery (54) and switch (56).

8. An apparatus according to claim 2 or 3 in which said electrical signal source (72) is part of a mechanical respirator (70), and in which the "on" signal is generated during the respirator's inhalation cycle, and the "off" signal is generated during the respirator's exhalation cycle.

9. An apparatus according to claim 8 additionally including a means (52) for attaching the housing (40) to said mechanical respirator (70) such that the gas from the respirator (70) will pass through the housing (40) during the respirator's inhalation cycle and carry the generated aerosol into the respirator (70).

10. An apparatus according to claim 1 in which said housing (40) additionally includes a temperature bath (34) for bringing the liquid medicine to be nebulized to, and maintaining said liquid medicine at, a constant temperature.

11. An apparatus according to claim 1 additionally comprising a means (36) for draining said temperature bath (34).

12. A method for providing an aerosol using a single source of compressed gas to generate the aerosol and an external electrical signal generator capable of generating a first and a second electrical signal to control the generation of said aerosol, said method comprising the steps of:

    directing said compressed gas to an aerosol transducer (22) attached to a liquid medicine reservoir (26) by a feed tube (24) when it is desired to produce an aerosol; and

    stopping the flow of compressed gas to the aerosol transducer (22) when it is desired to stop the production of aerosol;

    said compressed gas being directed to the aerosol transducer (22) through an electrically operated solenoid valve (6) which has two positions, a first position which opens a passageway between the compressed gas and the aerosol transducer (22), and a second position which closes the passageway between the compressed gas and the aerosol transducer (22), said valve (6) being electrically connected so as to detect the first and second signals generated by the external electrical signal source (72);

wherein when said first electrical signal is generated it is received by said valve (6) which moves to said first position, permitting said compressed gas to flow to said aerosol transducer (22) to generate an aerosol;

wherein when said second electrical signal is generated it is received by said valve (6) which moves to said second position, shutting off the flow of compressed gas to the aerosol transducer (22), terminating the generation of aerosol, **characterized in that**

said valve (6) when in its second position opens a passageway between the compressed gas and a turbine driven mixer (16) for mixing the liquid medicine in the reservoir (26), and when the first signal is received by the valve (6), the passageway between the compressed gas and the mixer (16) is closed during the generation of aerosol.

13. A method according to claim 12 in which said first electrical signal is an "on" signal, and the second electrical signal is an "off" signal.

14. A method according to claim 12 in which said first electrical signal is an "off" signal, and the second electrical signal is an "on" signal.

15. A method according to claim 12 additionally including the step of controlling the temperature of the liquid medicine in the reservoir (26).

16. A method according to claims 13 or 14 in which the external electrical signals are generated by a mechanical respirator (70) which generates the first signal during the respirator's inhalation cycle and which generates the second signal during the respirator's exhalation cycle, additionally including the step of directing the aerosol generated into the airstream of the respirator.

**Patentansprüche**

1. Vorrichtung zum Synchronisieren der Zerstäubung eines flüssigen Medikaments mit einer externen elektrischen Signalquelle, die ein erstes und ein zweites elektrisches Signal erzeugen kann, wobei die Vorrichtung aufweist:

ein Gehäuse (40), welches ein Reservoir (26) zum Bereithalten eines flüssigen, zu zerstäubenden Medikaments enthält;

einen Aerosol-Wandler (22) zum Zerstäuben des flüssigen Medikaments;

eine einzelne Quelle für Druckgas; und

eine Leitung (12) zum Anschluß des Druckgases an den Aerosol-Wandler (22);

wobei der Aerosol-Wandler (22) durch die Leitung (12) an ein elektrische betätigtes Magnetventil (6) angeschlossen ist, das an einer Druckgasleitung (4) angebracht ist;

wobei das Magnetventil (6) zwei Einstellungen hat, die erste Einstellung zum Verbinden der Druckgasleitung (4) und des Aerosol-Wandlers (22) durch die Leitung (12), wobei das Magnetventil (6) an die externe elektrische Signalquelle (72) zur Aufnahme des erzeugten und synchronisierten Signals angeschlossen ist, um sich in die erste Einstellung zu bewegen, wenn das erste elektrische Signal empfangen wird, und um sich in die zweite Einstellung zu bewegen, wenn das zweite elektrische Signal empfangen wird,

**dadurch gekennzeichnet, daß**

die zweite Einstellung des Magnetventils (6) zum Verbinden der Druckgasleitung (4) und eines turbinengetriebenen Mixers (16) durch eine Leitung (10) zum Umrühren des flüssigen, zu zerstäubenden Medikaments dient.

2. Vorrichtung nach Anspruch 1, bei der das erste elektrische Signal "Ein" und das zweite elektrische Signal "Aus" ist.

3. Vorrichtung nach Anspruch 1, bei der das erste elektrische Signal "Aus" und das zweite elektrische Signal "Ein" ist.

4. Vorrichtung nach Anspruch 1, wobei der turbinengetriebene Mixer (16) an dem Gehäuse außerhalb des Reservoirs (26) befestigt ist, um das flüssige, zu zerstäubende Medikament umzurühren.

5. Vorrichtung nach Anspruch 4, wobei der Mixer (16) weiterhin einen beweglichen Magneten (18), der an der Turbine (16) befestigt ist, und einen magnetischen Rührstab (28) enthält, der innerhalb des Reservoirs angeordnet ist und der mit dem beweglichen Magneten (18) zusammenwirkt und durch dessen Bewegung bewegt wird.

6. Vorrichtung nach Anspruch 1, wobei das Druckgas einen Druckbereich von bis zu etwa 345 kPa (50 psig) einnimmt.

7. Vorrichtung nach Anspruch 1, wobei die elektrische Signalquelle eine Batterie (54) und einen Schalter (56) aufweist.

**8.** Vorrichtung nach Anspruch 2 oder 3, bei der die elektrische Signalquelle (72) Teil eines mechanischen Respirators (70) ist und bei der das "Ein"-Signal während des Einatmungszyklus des Respirators und das "Aus"-Signal während des Ausatmungszyklus des Respirators erzeugt wird.

**9.** Vorrichtung nach Anspruch 8, welche zusätzlich Mittel (52) zum Anschluß des Gehäuses (40) an den mechanischen Respirator (70) in der Weise aufweist, daß das Gas vom Respirator 70 während des Einatmungszyklus des Respirators durch das Gehäuse (40) strömt und das erzeugte Aerosol in den Respirator (70) trägt.

**10.** Vorrichtung nach Anspruch 1, in welcher das Gehäuse (40) zusätzlich ein Temperaturbad (34) aufweist, um das flüssige, zu zerstäubende Medikament auf eine konstante Temperatur zu bringen und das flüssige Medikament auf der konstanten Temperatur zu halten.

**11.** Vorrichtung nach Anspruch 1, welche zusätzlich Mittel (36) zum Ablassen des Temperaturbades (34) aufweist.

**12.** Verfahren zur Erzeugung eines Aerosols mit Hilfe einer einzelnen Druckgasquelle, um das Aerosol zu erzeugen, und eines externen elektrischen Signalgenerators, der ein erstes und ein zweites elektrisches Signal erzeugen kann, um die Erzeugung des Aerosols zu steuern, wobei das Verfahren die Schritte aufweist:

Leiten des Druckgases zu einem Aerosol-Wandler (22), der durch ein Zufuhrrohr (24) mit einem Reservoir (26) für flüssiges Medikament verbunden ist, wenn ein Aerosol erzeugt werden soll;

Stoppen des Druckgasflusses zu dem Aerosol-Wandler (22), wenn die Erzeugung des Aerosols gestoppt werden soll;

wobei das Druckgas zu dem Aerosol-Wandler durch ein elektrisch betätigtes Magnetventil (6) geleitet wird, das zwei Einstellungen hat, eine erste Einstellung, die einen Durchgang zwischen dem Druckgas und dem Aerosol-Wandler (22) öffnet, und eine zweite Einstellung, die den Durchgang zwischen dem Druckgas und dem Aerosol-Wandler (22) schließt, wobei das Ventil (6) elektrisch so angeschlossen ist, daß es das erste und zweite von der externen elektrischen Signalquelle (72) erzeugte Signal nachweist;

wobei, wenn das erste elektrische Signal erzeugt wird, dieses von dem Ventil (6) aufgenommen wird, das sich in die erste Einstellung bewegt, was das Druckgas zu dem Aerosol-Wandler (22) fließen läßt, um ein Aerosol zu erzeugen;

wobei, wenn das zweite elektrische Signal erzeugt wird, dieses von dem Ventil (6) aufgenommen wird, das sich in die zweite Einstellung bewegt, was den Durchfluß von Druckgas zu dem Aerosol-Wandler (22) schließt und die Erzeugung von Aerosol beendet;

**dadurch gekennzeichnet, daß**

das Ventil (6), wenn es in der zweiten Einstellung ist, einen Durchgang zwischen dem Druckgas und einem turbinengetriebenen Mixer (16) öffnet, um das flüssige Medikament in dem Reservoir (26) umzurühren, und, wenn das erste elektrische Signal von dem Ventil (6) aufgenommen wird, der Durchgang zwischen dem Druckgas und dem Mixer (16) während der Erzeugung von Aerosol geschlossen wird.

**13.** Verfahren nach Anspruch 12, bei welchem das erste elektrische Signal ein "Ein"-Signal und das zweite elektrische Signal ein "Aus"-Signal ist.

**14.** Verfahren nach Anspruch 12, bei welchem das erste elektrische Signal ein "Aus"-Signal und das zweite elektrische Signal ein "Ein"-Signal ist.

**15.** Verfahren nach Anspruch 12, welches als weiteren Schritt die Steuerung der Temperatur des flüssigen Medikaments in dem Reservoir (26) enthält.

**16.** Verfahren nach Anspruch 13 oder 14, bei welchem die externen elektrischen Signale durch einen mechanischen Respirator erzeugt werden, der das erste elektrische Signal während des Einatmungszyklus des Respirators und das zweite elektrische Signal während des Ausatmungszyklus des Respirators erzeugt, und welches Verfahren zusätzlich den Schritt enthält, das erzeugte Aerosol in den Luftstrom des Respirators zu leiten.

**Revendications**

**1.** Appareil pour synchroniser la nébulisation d'un médicament liquide avec une source de signal électrique externe capable de générer un premier et un second signal électrique, ledit appareil comprenant :

un bâti (40) contenant un réservoir (26) pour garder le médicament liquide à nébuliser ;

un transducteur d'aérosol (22) pour nébuliser ledit médicament liquide ;

une seule source de gaz comprimé et

un conduit (12) pour relier le gaz comprimé au transducteur d'aérosol (22),

ledit transducteur d'aérosol (22) étant relié par un conduit (12) à une vanne magnétique électrique (6) qui est fixée au conduit (4) de gaz comprimé,

ladite vanne magnétique (6) ayant deux positions, la première position pour relier le conduit de gaz comprimé (4) et le transducteur d'aérosol (22) par le conduit (12), ladite vanne magnétique (6) étant fixée à ladite source de signal électrique externe (72) pour recevoir le signal généré et synchronisé pour se déplacer vers la première position lorsque le premier signal électrique est reçu et pour se déplacer vers la seconde position lorsque le second signal électrique est reçu,

**caractérisé en ce**

que la seconde position de ladite vanne magnétique (6) est pour relier le conduit de gaz comprimé (4) et un mélangeur à turbine (16) par un conduit (10) pour mélanger le médicament liquide à nébuliser.

2. Appareil selon la revendication 1 dans lequel le premier signal électrique est "en marche" et le second signal électrique est "à l'arrêt".

3. Appareil selon la revendication 1 dans lequel le premier signal électrique est "à l'arrêt" et le second signal électrique est "en marche".

4. Appareil selon la revendication 1 en plus dans lequel le mélangeur à turbine (16) est fixé au bâti à l'extérieur dudit réservoir (26) pour mélanger le médicament liquide qui doit être nébulisé.

5. Appareil selon la revendication 4 dans lequel ledit mélangeur (16) comprend en plus un aimant mobile (18) fixé à ladite turbine (16) et un bras mélangeur magnétique (28) situé à l'intérieur dudit réservoir qui coopère avec et qui est déplacé par le mouvement dudit aimant mobile (18).

6. Appareil selon la revendication 1 dans lequel ledit gaz comprimé a une pression maximum de 345 kPA (pression de 50 psi).

7. Appareil selon la revendication 1 dans lequel ladite source de signal électrique comprend une pile (54) et un commutateur (56).

8. Appareil selon la revendication 2 ou 3 dans lequel ladite source de signal électrique (72) est une partie d'un respirateur mécanique (70) et dans lequel le signal "en marche" est généré pendant le cycle d'inhalation du respirateur et le signal "à l'arrêt" est généré pendant le cycle d'exhalation du respirateur.

9. Appareil selon la revendication 8 comprenant de plus un organe (52) pour fixer le bâti (40) au respirateur mécanique mentionné (70) de telle manière que le gaz provenant du respirateur (70) traverse le bâti (40) pendant le cycle d'inhalation du respirateur et porte l'aérosol généré dans le respirateur (70).

10. Appareil selon la revendication 1 dans lequel ledit bâti (40) comprend de plus un bain de température (34) pour amener le médicament liquide à nébuliser à une température constante et maintenir ledit médicament liquide à cette température constante.

11. Appareil selon la revendication 1 comprenant de plus un organe (36) pour drainer ledit bain de température (34).

12. Procédé pour créer un aérosol utilisant une seule source de gaz comprimé pour générer l'aérosol et un générateur de signal électrique externe capable de générer un premier et un second signal électrique pour commander la génération dudit aérosol, ledit procédé comprenant les étapes de

diriger ledit gaz comprimé vers un transducteur d'aérosol (22) fixé à un réservoir de médicament liquide (26) par un tube d'alimentation (24) lorsque l'on désire produire un aérosol et

d'arrêter le débit de gaz comprimé vers le transducteur d'aérosol (22) lorsque l'on désire arrêter la production d'aérosol,

ledit gaz comprimé étant dirigé vers le transducteur d'aérosol (22) par une vanne magnétique (6) actionnée électriquement qui a deux positions, une première position qui ouvre un passage entre le gaz comprimé et le transducteur d'aérosol (22) et une seconde position qui ferme le passage entre le gaz comprimé et le transducteur d'aérosol (22), ladite valve (6) étant reliée électriquement de manière à détecter les premier et second signaux générés par la source de signal électrique externe (72), dans lequel ledit premier signal électrique généré est reçu par ladite valve (6) qui se déplace vers ladite première position en permettant au gaz comprimé sus-mentionné de s'écouler vers ledit transducteur d'aérosol (22) pour générer un aérosol,

dans lequel ledit second signal électrique généré est reçu par ladite valve (6) qui se déplace vers ladite seconde position en arrêtant le débit de gaz comprimé vers le transducteur d'aérosol (22) en terminant la génération d'aérosol,

**caractérisé en ce**

que ladite valve (6), lorsqu'elle est dans la seconde position, ouvre un passage entre le gaz comprimé et un mélangeur à turbine (16) pour mélanger le médicament liquide dans le réservoir (26) et, quand le premier signal est reçu par la valve (6), que le passage entre le gaz comprimé et le mélangeur (16) est fermé pendant la génération de l'aérosol.

13. Procédé selon la revendication 12 dans lequel le premier signal électrique est "en marche" et le second signal électrique est "à l'arrêt".

14. Procédé selon la revendication 12 dans lequel le premier signal électrique est "à l'arrêt" et le second signal électrique est "en marche".

15. Procédé selon la revendication 12 comprenant en plus l'étape de régler la température du médicament liquide dans le réservoir (26).

16. Procédé selon les revendications 13 ou 14 dans lequel les signaux électriques externes sont générés par un respirateur mécanique (70) qui génère le premier signal pendant le cycle d'inhalation du respirateur et qui génère le second signal pendant le cycle d'exhalation du respiration, qui comprend en plus l'étape de diriger l'aérosol généré dans la veine d'air du respirateur.

FIG.1.

FIG.2.

EP 0 298 389 B1

FIG.3.

FIG.4.